(19) Europäisches Patentamt
European Patent Office
Office européen des brevets

(11) **EP 4 786 981 A1**

(12) **EUROPEAN PATENT APPLICATION**
published in accordance with Art. 153(4) EPC

(43) Date of publication:
**05.08.2026 Bulletin 2026/32**

(21) Application number: **24869452.3**

(22) Date of filing: **26.09.2024**

(51) International Patent Classification (IPC):
**G01N 33/543** (2006.01) **A61B 5/151** (2006.01)
**A61B 5/157** (2006.01) **G01N 33/48** (2006.01)
**G01N 33/50** (2006.01) **G01N 33/53** (2006.01)

(52) Cooperative Patent Classification (CPC):
**A61B 5/151; A61B 5/157; G01N 33/48;**
**G01N 33/50; G01N 33/53; G01N 33/543**

(86) International application number:
**PCT/JP2024/034349**

(87) International publication number:
**WO 2025/070573 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.09.2023 JP 2023163247**
**16.07.2024 JP 2024113244**

(71) Applicant: **Toppan Holdings Inc.**
**Tokyo 110-0016 (JP)**

(72) Inventors:
• **TSUKAMOTO Kei**
**Tokyo 110-0016 (JP)**
• **SASAKI Nobuo**
**Tokyo 110-0016 (JP)**
• **UEMURA Daizo**
**Tokyo 110-0016 (JP)**
• **NAKAYAMA Masato**
**Tokyo 110-0016 (JP)**

(74) Representative: **Plasseraud IP**
**104 Rue de Richelieu**
**CS92104**
**75080 Paris Cedex 02 (FR)**

(54) **INSPECTION DEVICE AND INSPECTION METHOD**

(57)     This examination device is provided with:
a substrate (2) a microneedle (3) that pierces the skin to collect a body fluid; a body fluid flow path (4) that transfers the body fluid collected by the microneedle (3); and an immunoassay unit (5) that detects, using an antigen-antibody reaction, a biomarker that may be included in the body fluid that has been transferred through the body fluid flow path (4),
wherein the microneedle (3), the body fluid flow path (4), and the immunoassay unit (5) are disposed on a surface (2a) on one side of the substrate (2).

FIG.1

## Description

[Technical Field]

The present invention relates to an examination device and an examination method using an immunoassay method.

This application is based on and claims the benefit of priority from earlier Japanese Patent Application No. 2023-163247 filed in Japan on September 26, 2023, and Japanese Patent Application No. 2024-113244 filed in Japan on July 16, 2024, the descriptions of which are incorporated herein by reference.

[Background Art]

Conventionally, an examination device is known that obtains a body fluid, which may contain a biomarker indicating the presence or absence, or progression level of a disease, the presence or absence of pregnancy, or the like, from within a living body, and then performs diagnosis using an immunoassay method utilizing an antigen-antibody reaction.

For example, PTL 1 describes an examination device capable of collecting a body fluid by piercing a living body surface with microneedles arranged on a surface on one side of a substrate.

According to such an examination device, after the collected body fluid is transferred to another side, the biomarker in the body fluid can be detected using an antigen-antibody reaction while the body fluid is radially spread.

[Citation List]

[Patent Literature]

[PTL 1] WO 2019/118420

[Summary of the Invention]

[Technical Problem]

However, in the examination device of PTL 1, the microneedles are arranged on the surface on one side of the substrate, and the immunoassay unit that performs the antigen-antibody reaction is arranged on another surface of the substrate.

For this reason, during manufacturing, it is necessary to form the microneedles or the immunoassay unit on both the front and back surfaces of the substrate, which requires at least two special molds. As a result, the manufacturing cost of the examination device becomes high.

The present invention has been made in view of such circumstances, and an object thereof is to provide an examination device capable of reducing manufacturing costs, and an examination method using the examination device.

[Solution to Problem]

The present disclosure provides an examination device for the detection of a biomarker.

In a specific embodiment, the following aspects may be provided.

[1] An examination device including:

a substrate;
a microneedle that pierces skin to collect a body fluid;
a body fluid flow path that transfers the body fluid collected by the microneedle; and
an immunoassay unit that detects, using an antigen-antibody reaction, a biomarker that may be included in the body fluid that has been transferred through the body fluid flow path,
wherein the microneedle, the body fluid flow path, and the immunoassay unit are disposed on a surface on one side of the substrate.

[2] The examination device according to [1], wherein a microstructure for improving wettability of the body fluid flow path is formed on a surface of the body fluid flow path.

[3] The examination device according to [2], wherein the microstructure is a groove extending in an extending direction of the body fluid flow path.

[4] The examination device according to [2] or [3], wherein a plurality of the grooves extend parallel to each other on a surface of the body fluid flow path.

[5] The examination device according to [3] or [4], wherein the number of the grooves increases toward a downstream side of the body fluid flow path.

[6] The examination device according to [4] or [5], wherein a connecting groove is formed that connects the plurality of grooves extending in the extending direction of the body fluid flow path to each other.

[7] The examination device according to any one of [3] to [6], wherein

a width of the groove is in a range of 100 nm or more and less than 1,000 nm.

[8] The examination device according to any one of [1] to [7], wherein

at least a portion of the body fluid flow path has a shape in which a width increases toward a downstream side.

[9] The examination device according to any one of [1] to [8], wherein the body fluid flow path has a plurality of repeating units having the shape, that are connected in the extending direction of the body fluid flow path.

[10] The examination device according to any one of [1] to [9], wherein at least a portion of the body fluid flow path has a depth that becomes deeper toward a downstream side.

[11] The examination device according to [2], wherein the microstructure includes a plurality of microprotrusions provided on the body fluid flow path.

[12] The examination device according to [11], wherein the microprotrusions have a substantially conical shape, and when a height of the microprotrusions is a, a width dimension of a bottom surface portion of the microprotrusions is b, and an arrangement interval of the microprotrusions is c, the microprotrusions have a shape that satisfies the following formulas (1), (2), and (3).

$$b \leq 600 \ (\text{unit: nm}) \ ... \ (1)$$

$$a/b \geq 0.3 \ ... \ (2)$$

$$200 \leq c \leq 400 \ (\text{unit: nm}) \ ... \ (3)$$

[13] The examination device according to any one of [1] to [12], wherein unevenness is formed on a surface of the microneedle.

[14] The examination device according to any one of [1] to [12], wherein a protective film is provided on a tip end side of the microneedle, and a thickness of the protective film is less than a height of the microneedle.

[15] The examination device according to any one of [1] to [14], wherein a section of the substrate located on another side of the immunoassay unit is formed to be transparent.

[16] The examination device according to [15], wherein a magnifying optical system is provided on another side of the section located on the other side of the immunoassay unit.

[17] The examination device according to any one of [1] to [16], wherein the surface on the one side of the substrate and a surface of the microneedle are formed of the same material, and a water contact angle on the surface on the one side is 45° or less.

[18] The examination device according to any one of [1] to [17], wherein the microneedle is a solid, hollow, porous, or hydrogel structure.

[19] The examination device according to any one of [1] to [18], wherein the body fluid flow path has a shape that surrounds the microneedle.

[20] The examination device according to any one of [1] to [18], wherein the immunoassay unit is provided with a test unit that visualizes a presence of the biomarker in the body fluid, and the test unit is provided with a plurality of color-producing regions that visualize a presence of mutually different biomarkers.

[21] The examination device according to [20], wherein the color-producing regions are formed in a region on an inner side of a groove structure formed on a surface of the body fluid flow path and extending in a closed loop shape, and antibodies that specifically capture mutually different biomarkers are provided in each of the plurality of color-producing regions.

[22] An examination method, comprising: a step of piercing skin to collect a body fluid with a microneedle of an examination device, the examination device being provided with, on a surface on one side of a substrate, the microneedle, a body fluid flow path that transfers the body fluid, and an immunoassay unit that detects a biomarker using an antigen-antibody reaction;

a step of transferring the collected body fluid through the body fluid flow path; and a step of detecting, using the immunoassay unit, a biomarker contained in the body fluid that has been transported.

[23] The examination method according to [22], wherein the immunoassay unit is provided with a test unit that visualizes a presence of the biomarker in the body fluid, the test unit is provided with a plurality of color-producing regions that visualize a presence of mutually different biomarkers, the color-producing regions are formed in a region on an inner side of a groove structure formed on a surface of the body fluid flow path and extending in a closed loop shape, antibodies that specifically capture mutually different biomarkers are provided in each of the plurality of color-producing regions, and different biomarkers are captured and detected in each color-producing region by the antibodies.

[Advantageous Effects of the Invention]

According to the present invention, because the microneedle and the immunoassay unit are integrated on the surface on one side of the substrate, the number of special molds for the substrate can be reduced. Therefore, it is possible to provide an examination device capable of reducing manufacturing costs, and an examination method using the examination device.

[Brief Description of the Drawings]

Fig. 1 is a schematic bottom view of an examination device according to a preferable embodiment of the present invention.
Fig. 2 is a vertical cross-sectional view of the examination device taken along line Y-Y shown in Fig. 1.
Fig. 3 is a schematic side view of the vicinity of the microneedles of the examination device shown in Fig. 1.
Fig. 4 is a cross-sectional view in the vicinity of microprotrusions according to a modification, taken along a plane passing through the center of the microprotrusions.
Fig. 5 is a schematic bottom view of a second downstream portion according to a second antibody fixing method.
Fig. 6 is a schematic cross-sectional view of the vicinity of the surface of a second downstream portion according to a third antibody fixing method.
Fig. 7 is a schematic side view showing a method of forming microprotrusions according to a fifth antibody fixing method.
Fig. 8A is an enlarged side view of a second downstream portion according to a sixth antibody fixing method.
Fig. 8B is an enlarged bottom view of the second downstream portion according to the sixth antibody fixing method.
Fig. 9A is an enlarged side view of a second downstream portion according to a seventh antibody fixing method.
Fig. 9B is an enlarged bottom view of the second downstream portion according to the seventh antibody fixing method.
Fig. 10 is a schematic bottom view of a body fluid flow path according to another preferable embodiment of the present invention.
Fig. 11 is a schematic diagram showing the function of a body fluid flow path according to yet another preferable embodiment of the present invention.
Fig. 12 is a schematic perspective view of the vicinity of a body fluid flow path of an examination device according to another preferable embodiment of the present invention.
Fig. 13 is a schematic bottom view of the vicinity of a transfer groove shown in Fig. 12.
Fig. 14A is a schematic side view showing the vertical position of a substrate in a standard state of an examination device according to still another preferable embodiment of the present invention.
Fig. 14B is a schematic side view showing the vertical position of the substrate of the examination device shown in Fig. 14A in a piercing state.
Fig. 15 is a schematic bottom view of the vicinity of a test unit of an examination device capable of multi-item detection according to an embodiment of the present invention.
Fig. 16A is a schematic side view showing microneedles on which horizontal grooves extending in the circumferential direction are formed.
Fig. 16B is a schematic side view showing microneedles formed in an arrowhead shape.
Fig. 17 is a high-magnification image of a mold structure obtained by etching treatment, having a pore period of 200 nm, a pore diameter of 150 nm, and a pore length of about 400 nm.
Fig. 18 is a high-magnification image of a microstructure including the pillars of Example 1.
Fig. 19 is a high-magnification image of a mold structure obtained by etching treatment, having a pore period of 200 nm, a pore diameter of 150 nm, and a pore length of about 1 $\mu$m.
Fig. 20 is a high-magnification image of a microstructure including the pillars of Example 2.
Fig. 21 is a top view of a flow path prototype of Example 3.

[Description of the Embodiments]

Hereinafter, a preferable embodiment of the present invention will be described in detail with reference to the accompanying drawings in some cases.

[Examination Device]

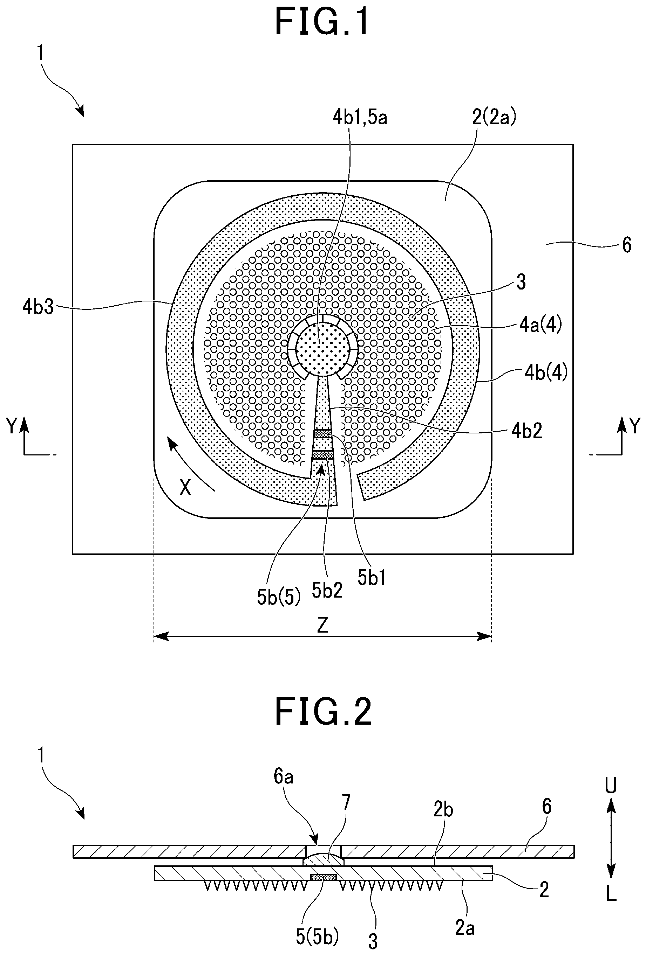

Fig. 1 is a schematic bottom view of an examination device 1 according to a preferable embodiment of the present invention, and Fig. 2 is a vertical cross-sectional view of the examination device 1 taken along line Y-Y shown in Fig. 1.

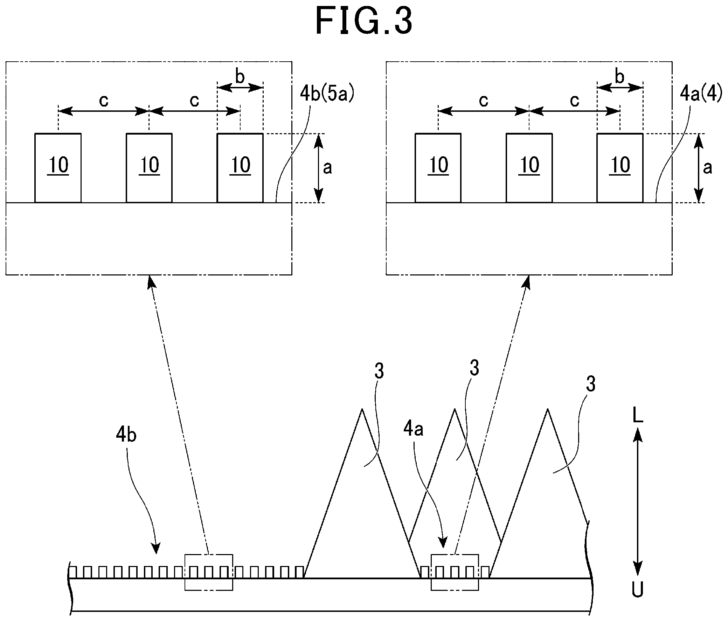

Furthermore, Fig. 3 is a schematic side view of the vicinity of the microneedles of the examination device 1 shown in Fig. 1.

The examination device 1 includes a substantially flat plate-shaped substrate 2, a plurality of microneedles 3 and a body fluid flow path 4 formed on a surface 2a on one side of the substrate 2, an immunoassay unit 5 provided in a portion of the body fluid flow path 4, and a fixing seal 6 and an optical system 7 that enhances the visibility of detection results, which are arranged on the other side of the substrate 2. In the following description, the one side of the substrate 2 is referred to as "lower" ("L" in the drawings), and the opposite side (the other side) is referred to as "upper" ("U" in the drawings).

The examination device 1 is a device that collects body fluid by piercing the skin with the microneedles 3, and visualizes whether a biomarker, which indicates the presence or absence, or progression level of a disease, the presence or absence of pregnancy, or the like, is contained in the body fluid.

The size of the substrate 2 is not particularly limited, but the width Z, being the larger dimension among the depth and width of the substrate 2, is preferably 1 cm or more and 3 cm or less, and particularly preferably 1 cm or more and 2 cm or less.

The substrate 2 is preferably formed of a biocompatible material. Examples of biocompatible materials include thermoplastic resins such as medical silicone, polyglycolic acid, and cyclic olefin copolymer. The substrate 2 may be a laminate composed of a plurality of layers formed of different materials, but it is preferable to use a single material from the viewpoint of suppressing manufacturing costs.

The fixing seal 6 is formed to be wider than the substrate 2 in a bottom view. An adhesive layer is provided on the lower surface of the fixing seal 6, and no adhesive layer is provided on the upper surface. By adhering the lower surface of a section of the fixing seal 6 that does not overlap with the substrate 2 in a bottom view to the skin, the microneedles 3 arranged on the lower surface 2a of the substrate 2 can be fixed in a state of piercing the skin.

A through-hole 6a that penetrates in a vertical direction is formed in the section of the fixing seal 6 located above the optical system 7, allowing the optical system 7 to be viewed from above through the through-hole 6a.

Each microneedle 3 preferably has a conical shape or polygonal pyramid shape, but is not limited to having such a shape. The microneedle 3 may also have a shape in which a columnar (such as a circular columnar-shaped or polygonal columnar-shaped) base section and a conical or polygonal pyramid tip end section are joined.

The height of each microneedle 3 is preferably in a range of 100 $\mu$m or more and 2 mm or less, and more preferably in a range of 800 $\mu$m or more and 1 mm or less. As a result of setting the height of the microneedles 3 in such a range, the microneedles 3 can pierce up to the vicinity of the boundary between the epidermis and the dermis, and interstitial fluid (an example of body fluid) can primarily be collected, which enables a minimally invasive examination device 1 to be realized compared to a case where blood is also collected.

However, the height of the microneedles 3 is not particularly limited, and the height of the microneedles 3 may be set to a height that reaches a depth capable of collecting not only interstitial fluid but also blood from within the body. Hereinafter, the microneedles 3 may also be simply referred to as "needles 3".

The angle of the tip end of the microneedles 3 is not particularly limited, but is preferably in a range of $60° \pm 40°$.

The microneedles 3 and the body fluid flow path 4 are preferably formed of the same material as the substrate 2. The overall materials of the microneedles 3, body fluid flow path 4, and substrate 2 are not particularly limited, but are preferably integrally and continuously molded from a biocompatible material such as polyglycolic acid, polycarbonate, cyclic olefin copolymer (COC), cyclic olefin polymer (COP), polyether ether ketone (PEEK), alginate, chitin, chitosan, collagen, hydroxypropyl cellulose, pullulan, or the like. Examples of the molding method include injection molding, thermal compression molding, casting, and the like.

The surface of the needle 3 and the lower surface 2a of the substrate 2 are each formed with hydroxyl groups and fine unevenness due to oxygen plasma treatment (hydrophilic processing).

For this reason, the hydrophilicity of these portions is extremely high, and the water contact angle on the lower surface 2a of the substrate 2 including the microneedles 3 and the body fluid flow path 4 is 45° or less. Therefore, the interstitial fluid collected by the microneedles 3 can be smoothly transferred to the body fluid flow path 4.

Examples of methods that cause the water contact angle on the lower surface 2a of the substrate 2 to become 45° or less include, in addition to plasma treatment using oxygen or the like, corona treatment, flame treatment, chemical treatment, and UV treatment, but are not limited to such method.

Furthermore, the microneedles 3 and the body fluid flow path 4 may be integrally formed with the substrate 2 as in this embodiment, but after forming the substrate, the substrate itself may be processed to form the microneedles and the body fluid flow path, or members forming the microneedles and the body fluid flow path that have been formed separately from the substrate may be attached to the surface of the substrate on one side.

Each microneedle 3 has a solid structure, but the structure of the microneedle is not limited to a solid structure, and may be a hollow structure, a porous structure, or a hydrogel structure. As a result making the microneedle a hollow, porous, or hydrogel structure, the wettability (hydrophilicity) of the microneedle can be improved.

The collection of body fluid by the microneedle 3 refers to adhering the body fluid to the surface of the microneedle 3, or in the case of a hollow needle 3, accommodating the body fluid inside the needle 3.

When the microneedle has a hollow structure, for example, by forming a through-hole that penetrates upward from the tip end of the microneedle and connects to the body fluid flow path, body fluid such as interstitial fluid can be collected through the through-hole.

The microneedles 3 are arranged in a region surrounded by the body fluid flow path 4 on the lower surface 2a of the substrate 2.

The body fluid flow path 4 is capable of receiving interstitial fluid from the microneedles 3 and then transporting the interstitial fluid from an upstream side section of the body fluid flow path 4 toward a downstream side. The body fluid flow path 4 includes a needle peripheral portion 4a located on the upstream side and extending through the gaps between the microneedles 3, and a downstream portion 4b located downstream of the needle peripheral unit 4a.

The needle peripheral portion 4a is a flow path whose upper portion is defined by the lower surface 2a of the substrate 2 around the microneedles 3.

The downstream portion 4b includes a first downstream portion 4b1 located substantially in the center of the region where the plurality of microneedles 3 are arranged on the lower surface 2a of the substrate 2, a second downstream portion 4b2 extending substantially linearly in the horizontal direction from the first downstream portion 4b1 to the vicinity of the edge of the lower surface 2a of the substrate 2, and a third downstream portion 4b3 having a shape that surrounds the microneedles 3 from the downstream end portion (the end portion on the edge side of the substrate 2) of the second downstream portion 4b2. The first downstream portion 4b1 plays the role of collecting the interstitial fluid, collected from the living body by the microneedles 3, at a central portion of the substrate 2. The third downstream portion 4b3 plays the role of exclusively transporting the interstitial fluid to the downstream side, and corresponds to a water absorption portion of a conventional examination device using an immunoassay method.

The downstream portion 4b is formed by upwardly recessing the lower surface 2a of the substrate 2, but it is not always necessary to form the downstream portion 4b in such a way. Further, the height position of the surface of the downstream portion 4b may be higher than, lower than, or at the same height position as a base portion (the end portion on the substrate 2 side) of the microneedles 3.

As shown in FIG. 3, a plurality of microprotrusions 10 protruding downward are formed in the needle peripheral portion 4a. The microprotrusions 10 can be formed by, for example, injection molding, nanoimprint lithography (NIL), or the like, but the method of forming the microprotrusions 10 is not limited to such a method. The microprotrusions 10 are an example of a "microstructure" for increasing the surface area of the body fluid flow path 4 and improving the wettability of the needle peripheral portion 4a. Each microprotrusion 10 has a substantially circular columnar shape.

It is preferable that the lateral width b of the end portion of the microprotrusions 10 on the substrate 2 side is 600 nm or less (more preferably 360 nm or less), and the dimensional ratio of the height a of the microprotrusions 10 to the lateral width b is 0.3 or more (more preferably 0.5 or more) ($a/b \geq 0.3$, $a/b \geq 0.5$). Furthermore, it is preferable that the arrangement interval c between the microprotrusions 10 is set in a range of 200 nm or more and 400 nm or less. In the magnified views in Fig. 3, "c" represents the horizontal distance between the central axes of the microprotrusions 10.

As a result of configuring the microprotrusions 10 in this way, the surface area of the needle peripheral portion 4a can be increased. For this reason, the needle peripheral portion 4a can be maintained in a highly hydrophilic state with a wet area ratio of 70% or more and a contact angle of 45° or less over a long period.

Similarly, a plurality of microprotrusions 10 are also formed on the surface (lower surface) of the downstream portion 4b. With respect to the microprotrusions 10 of the downstream portion 4b, as a result of setting the lateral width of the bottom surface, the ratio of the height to the lateral width, and the arrangement interval between the microprotrusions 10 in the above ranges, the downstream portion 4b can be maintained in a very highly hydrophilic state with a wet area ratio of 70% or more and a contact angle of 45° or less over a long period.

Note that, in the measurement of the wet area ratio of the needle peripheral portion 4a or the downstream portion 4b, a plate-shaped test piece is firstly prepared having a surface with the same surface state, such as the material, surface treatment, microprotrusion arrangement, and the like, as the needle peripheral portion 4a or the downstream portion 4b.

Next, in a state where the surface of the test piece is facing upward and horizontal, water is sprayed from a distance of 100 mm in several separate portions, in a total amount of 0.016 cc/cm². For example, if the surface is 100 cm², the amount of water sprayed is 1.6 cc.

Then, the surface is made to stand perpendicular to horizontal for 5 seconds, and the wet area ratio, which is the ratio of the wet area (area of the section wet with water) to the total area of the surface of the test piece, is determined. The wet area can be determined, for example, by analyzing an image obtained by photographing the surface of the test piece.

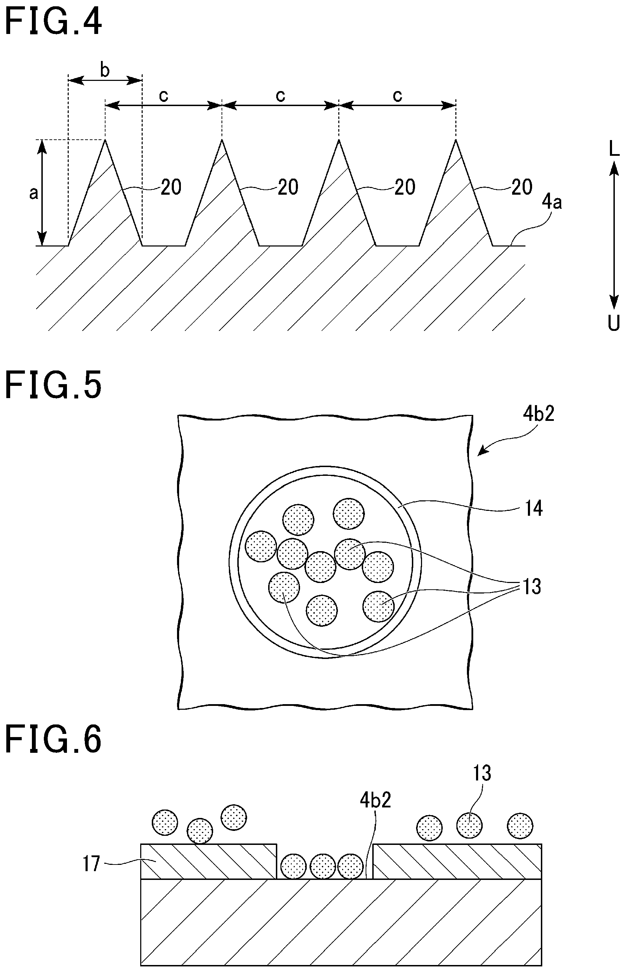

The shape of the microprotrusions formed in the body fluid flow path 4 is not particularly limited. For example, as shown in Fig. 4, the microprotrusions 20 may be formed in a conical shape similar to the microneedles 3. Similarly, in this case, to enhance the hydrophilicity of the body fluid flow path 4, it is preferable to set the lateral width b of the bottom surface portion (which makes contact with the body fluid flow path 4) of the microprotrusion 20 to a dimension of 600 nm or less (more preferably 360 nm or less), the dimensional ratio of the height a of the microprotrusion 20 to the lateral width b to 0.3 or more (more preferably 0.5 or more) (a/b $\geq$ 0.3, a/b $\geq$ 0.5), and the arrangement interval c between the microprotrusions 10 in a range of 200 nm or more and 400 nm or less.

As described above, by making the body fluid flow path 4 highly hydrophilic and forming the microprotrusions 10 and 20 over the entire body fluid flow path 4, a portion of the interstitial fluid collected by the microneedles 3 can be passively and smoothly transferred to the downstream side of the body fluid flow path 4.

At this time, due to the viscosity of the interstitial fluid, a flow of interstitial fluid is formed from the surface of the microneedles 3 and the needle peripheral portion 4a toward the downstream side of the body fluid flow path 4 (in the direction of arrow X indicating the extending direction of the body fluid flow path 4, see Fig. 1).

In conventional examination devices using an immunochromatography method, the body fluid flow path often uses a pad made of fibers such as cellulose.

In contrast, the entire body fluid flow path 4 of the present embodiment is subjected to surface treatment such as plasma treatment, corona treatment, flame treatment, chemical treatment, or UV irradiation.

For this reason, compared to the body fluid flow path of a conventional examination device, a situation where the transport of body fluid stagnates can be suppressed, and the controllability of body fluid transport can be improved.

In the downstream portion 4b of the body fluid flow path 4, the immunoassay unit 5 that detects a biomarker contained in the interstitial fluid using an antigen-antibody reaction is formed in the first downstream portion 4b1 and the second downstream portion 4b2.

As described above, because the microneedles 3, the body fluid flow path 4, and the immunoassay unit 5 are all arranged on the lower surface 2a (the surface on the one side) of the substrate 2, a special mold for the upper surface 2b of the substrate 2 can be omitted. Therefore, the manufacturing cost of the examination device 1 can be reduced.

In addition, by forming the third downstream portion 4b3 in a shape that surrounds the microneedles 3 as shown in Fig. 1, a sufficient length of the body fluid flow path 4 can be ensured even with a compact substrate 2. Therefore, even in a case where a biomarker contained in only a trace amount in the interstitial fluid is detected, a large amount of interstitial fluid can be supplied to the immunoassay unit 5, and the color production of the test unit 5b1, which will be described later, can be intensified.

The immunoassay unit 5 includes a conjugate unit 5a that labels the biomarker in the interstitial fluid, and a color-producing unit 5b that visualizes the detection result.

The conjugate unit 5a is formed in the first downstream portion 4b1. The conjugate unit 5a is provided with a labeling substance that specifically binds to and labels the biomarker to be detected.

The labeling substance has a binding portion that specifically binds to the target biomarker, and a labeling portion. As the binding portion, an antibody, a fragmented antibody, an aptamer, or the like can be used.

Examples of the labeling portion include metal colloid particles, fluorescent dye particles, colored latex particles, up-conversion phosphor particles, and quantum dot particles, but are not limited to such particles. The particle size of the labeling portion is preferably 20 nm or more and 1,000 nm or less.

When the interstitial fluid is collected from the microneedles 3 into the conjugate unit 5a, the labeling substance of the conjugate unit 5a disperses in the interstitial fluid. As a result, the biomarker that may be contained in the interstitial fluid binds to the labeling substance at the conjugate unit 5a or a position downstream thereof, and a primary reaction that causes the biomarker to become labeled occurs.

Subsequently, the biomarker to which the labeling substance is bound (hereinafter, also referred to as "complex") flows toward the downstream side in the body fluid flow path 4, and is visualized at the color- producing unit 5b (secondary reaction).

The color-producing unit 5b is formed in the second downstream portion 4b2. The color-producing unit 5b includes a test unit 5b1 provided with a first antibody capable of specifically capturing the biomarker to which the labeling substance is bound, and a control unit 5b2 arranged on the downstream side of the test unit 5b1.

Examples of the first antibody include an antibody, a fragmented antibody, and an aptamer, but are not limited thereto.

In a case where the interstitial fluid contains the biomarker, as the interstitial fluid reaches the immunoassay unit 5 and further flows through the third downstream portion 4b3 in the direction of arrow X, the complex is successively captured by the first antibody of the test unit 5b1.

As a result, the labeling substance is concentrated together with the biomarker in the test unit 5b1, and the presence or absence and intensity of reflected light, fluorescence, or the like, derived from the labeling substance can be confirmed visually or using a device. If the target biomarker is not contained in the collected interstitial fluid, the test unit 5b1 does not produce color. In this way, the test unit 5b1 can visualize the presence of the biomarker in the body fluid.

The control unit 5b2 is provided with a second antibody that specifically captures the labeling substance. Although the first antibody and the second antibody of the present embodiment are arranged between the plurality of microprotrusions 10 when fixed to the beads, as will be described in detail later with reference to Figs. 5 to 9B, the first antibody and the second antibody may be fixed to the surface (lower surface) of the second downstream portion 4b2, or directly fixed to the second downstream portion 4b2 or the microprotrusions 10 without using beads. Examples of the second antibody include an antibody, a fragmented antibody, and an aptamer, but are not limited thereto.

When the interstitial fluid is transported through the body fluid flow path 4 and reaches the control unit 5b2, the labeling substance dispersed in the interstitial fluid is captured by the second antibody.

As a result, the labeling substance is concentrated in the control unit 5b2, and the presence or absence and intensity of reflected light, fluorescence, or the like, derived from the labeling substance can be confirmed visually or using a device.

The operator of the examination device 1 can confirm whether the interstitial fluid, which is the sample, has been normally supplied to the color-producing unit 5b by checking the presence or absence of color production of the control unit 5b2. In a case where the control unit 5b2 is colored, the operator can determine that the interstitial fluid has been normally supplied to the color-producing unit 5b.

Here, the section of the substrate 2 located above the color-producing unit 5b is transparently formed.

In addition, an optical system 7 that enhances the visibility of the color-producing unit 5b, which indicates the detection result, is arranged above the transparent section of the substrate and the color-producing unit 5b.

For this reason, the operator of the examination device 1 can easily confirm the reflected light of the color-producing unit 5b from above the through-hole 6a of the fixing seal 6, through the optical system 7, with the naked eye or by using an imaging device such as a smartphone.

Examples of the optical system 7 that enhances visibility include a magnifying optical system such as a magnifying lens that magnifies the reflected light of the color-producing unit 5b, an optical system that prevents scattered light and/or reflected light, and an optical system that enhances contrast, but are not limited to such systems. Examples of the optical system that prevents scattered light include an optical system that reduces reflection and scattering of light as a result of being subjected to a coating applied to the surface of an optical material such as glass. For example, by forming an anti-reflection film (AR coating) on the surface of the optical material, light of a specific wavelength can be reduced, and the characteristics of the optical material can be improved. In this case, an improvement in contrast can also be expected.

Hereinafter, a method of fixing the antibody (the first antibody and the second antibody) to an arbitrary position on the surface (lower surface) of the second downstream portion 4b2 or the microprotrusions 10, which have been subjected to hydrophilic processing, will be described in detail. The antibody can be fixed directly to the surface of the second downstream portion 4b2 or the microprotrusions 10, or can be fixed via a support. The material of the support is not particularly limited, but can be composed of polymer particles such as beads or glass particles, for example. Note that the method of fixing the antibody is not limited to the following methods.

<Fixing to Surface of Second Downstream Portion 4b2>

(First Fixing Method)

The antibody or a support to which the antibody is fixed is embedded in a highly viscous gel, attached to an arbitrary region of the second downstream portion 4b2, and then made to adsorb. Specifically, the antibody or the support to which the antibody is fixed is mixed with a gel monomer, an arbitrary amount is then added to the surface of the second downstream portion 4b2 and left to stand until gelation occurs, which causes the antibody to become fixed to the second downstream portion 4b2.

(Second Fixing Method)

Fig. 5 is a schematic bottom view of a second downstream portion 4b2 according to a second antibody fixing method.

As shown in Fig. 5, a support 13 to which the antibody is fixed is added by inkjet or the like to the inside of a frame 14, which has been subjected to the hydrophobic treatment on the surface of the second downstream portion 4b2, and then dried to fix the antibody. Note that the antibody may be added without using the support 13.

The hydrophobic frame 14 may be formed, for example, as a fine groove by etching a portion of the hydrophilic second downstream portion 4b2 by laser processing, or may be formed by applying a hydrophobic composition to the second downstream portion 4b2.

For example, in a case where the hydrophobic frame 14 is formed in the test unit 5b1 and the support 13 to which the first antibody is fixed is fixed in the region inside the frame 14, when the interstitial fluid initially flows to the test unit 5b1 on the second downstream portion 4b2, the interstitial fluid passes around the frame 14 so as to bypass the frame 14. However, when the amount of interstitial fluid flowing through the test unit 5b1 per unit time increases, at a certain point, the interstitial fluid crosses the frame 14 and enters the hydrophilic region inside the frame 14. As a result, the complex in the interstitial fluid is captured by the first antibody and concentrated, and the inside of the frame 14 becomes colored.

As a result of arranging the antibody or the support 13 to which the antibody is attached inside the hydrophobic frame 14, it is possible to prevent the antibody from flowing downstream together with the interstitial fluid.

The diameter of the frame 14 may be about 150 $\mu$m, but the size of the frame 14 is not particularly limited.

In addition, in Fig. 5, the frame 14 has a circular shape in a bottom view, but the shape of the hydrophobic frame surrounding the first antibody is not particularly limited, and may be formed in another closed loop shape such as a rectangle, for example. A plurality of frames 14 may be provided side by side in a direction substantially orthogonal to the extending direction X of the body fluid flow path 4, such that the plurality of circular frames 14 look like a single line as a whole.

(Third Fixing Method)

Fig. 6 is a schematic cross-sectional view of the vicinity of the surface of a second downstream portion 4b2 according to a third antibody fixing method. As shown in Fig. 6, a mask 17 is attached to at least a region of the surface of the second downstream portion 4b2 where the antibody is not to be fixed, and the support 13 to which the antibody is fixed is added to the second downstream portion 4b2 and then dried to fix the antibody. The mask 17 is removed after the support 13 is fixed. Note that the antibody may be added without using the support 13.

(Fourth Fixing Method)

First, similarly to the third fixing method, after masking the region of the surface of the second downstream portion 4b2 where the antibody is not to be fixed, a binding molecule is added to the second downstream portion 4b2 and then dried. The binding molecule is a molecule capable of binding to the antibody or the support 13.

After drying, the mask is removed, and the antibody or the support 13 to which the antibody is fixed is added to the second downstream portion 4b2 and then dried, which enables the antibody to be fixed to the second downstream portion 4b2. Note that, after drying the antibody, the second downstream portion 4b2 may be washed by supplying a buffer solution or the like to remove the antibody that is not bound to the binding molecule.

<Fixing to Microprotrusions 10>

As a method for fixing the antibody to the microprotrusions 10, the first to fourth fixing methods described above can be adapted, and for example, the following fifth to seventh fixing methods can also be used.

(Fifth Fixing Method)

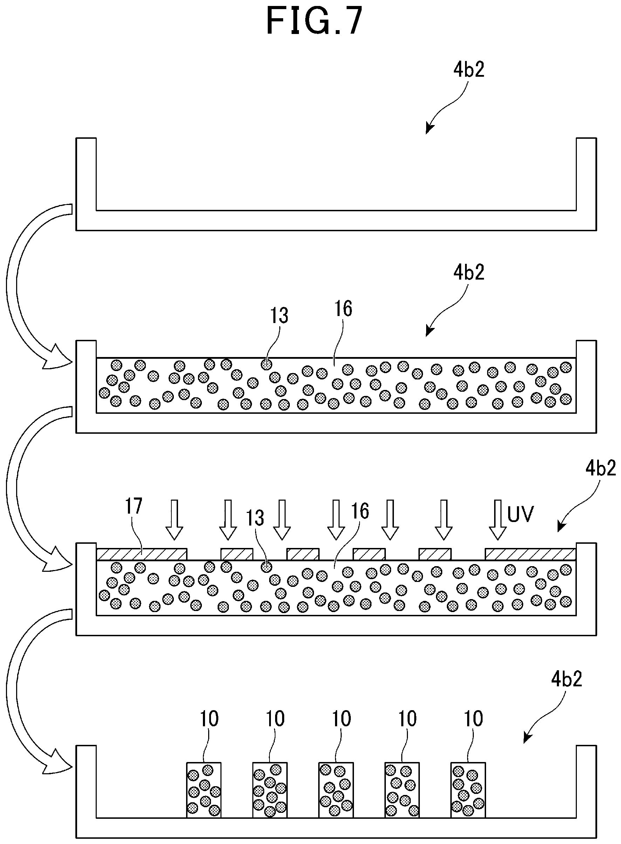

Fig. 7 is a schematic side view showing a method of forming microprotrusions 10 according to a fifth antibody fixing method.

As shown in Fig. 7, a photocurable resin 16 in which the antibody or the support 13 to which the antibody is fixed is dispersed is firstly added to the second downstream portion 4b2.

Then, a mask 17 used in photolithography is attached to the surface of the resin 16 to cover the section where the microprotrusions 10 are not to be formed, and light (ultraviolet light or the like) of a wavelength corresponding to the type of resin is irradiated to partially cure the photocurable resin 16.

Finally, the uncured photocurable resin 16 is removed by washing and then dried, which enables microprotrusions 10 with the antibody fixed to the surface to be obtained.

(Sixth Fixing Method)

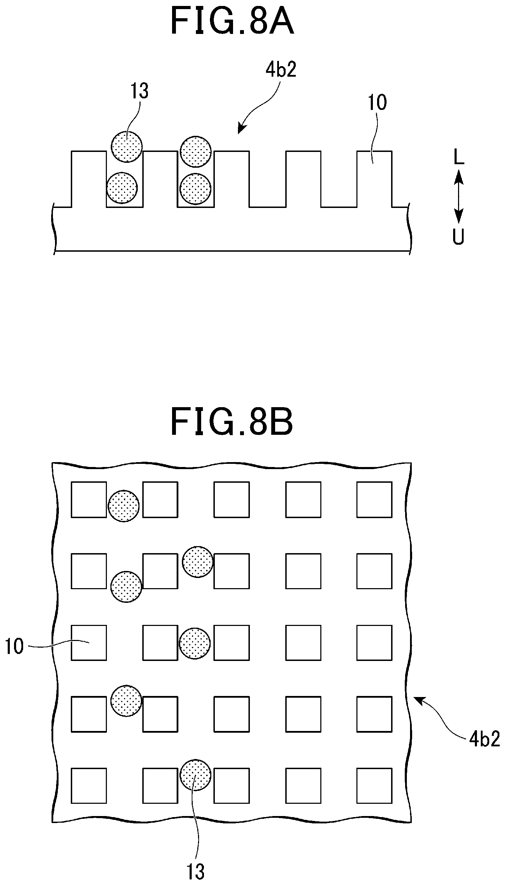

Fig. 8A is an enlarged side view of a second downstream portion 4b2 according to a sixth antibody fixing method, and Fig. 8B is an enlarged bottom view of a second downstream portion 4b2 according to the sixth antibody fixing method.

First, with the microprotrusions 10 formed by microfabrication such as injection molding or NIL facing upward, the support 13 to which the antibody is fixed is added to the vicinity of the microprotrusions 10 and left to stand for a certain period of time.

Then, the second downstream portion 4b2 is washed to remove the support 13 located outside the spaces between the microprotrusions 10, and then dried.

(Seventh Fixing Method)

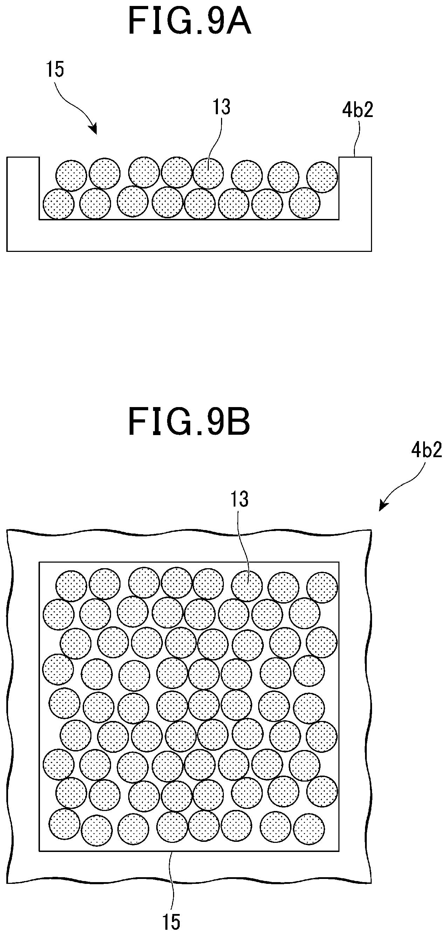

Fig. 9A is an enlarged side view of a second downstream portion 4b2 according to a seventh antibody fixing method, and Fig. 9B is an enlarged bottom view of a second downstream portion 4b2 according to the seventh antibody fixing method.

As shown in Fig. 9A, first, a well structure (recess portion) 15 is formed at an arbitrary position of the second downstream portion 4b2 by microfabrication such as injection molding or NIL.

Then, the support 13 to which the antibody is fixed is added to the vicinity of the well structure 15 and left to stand for a certain period of time.

Subsequently, the support 13 located outside the well structure 15 is removed by washing, and then drying is performed, which enables the antibody to be fixed at an arbitrary position of the second downstream portion 4b2.

On the other hand, as shown in Fig. 1, the second downstream portion 4b2 of the body fluid flow path 4, in which the color-producing unit 5b is formed, has a shape in which the width dimension increases toward the downstream side. As a result of forming the second downstream portion 4b2 of the body fluid flow path 4 in this way, the flow velocity of the interstitial fluid passively flowing through the immunoassay unit 5 can be increased compared to a case where the width dimension of the second downstream portion 4b2 is formed to be constant.

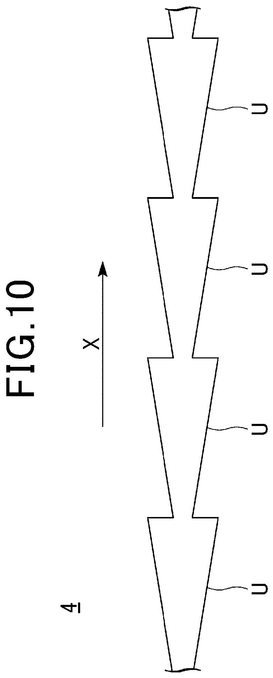

Furthermore, as shown in Fig. 10, the body fluid flow path 4 may be formed in a shape in which a plurality of repeating units U, each having a shape in which the width dimension increases toward the downstream side, are connected in the extending direction of the body fluid flow path (the direction X in which the body fluid flows). As a result of forming the body fluid flow path 4 in this way, the velocity of the interstitial fluid passively flowing through the body fluid flow path 4 can be further increased compared to a case where the width dimension simply continues to increase toward the downstream side.

In addition, in the present embodiment, the needle peripheral portion 4a is arranged around the microneedles 3, and the downstream portion 4b is arranged so as to surround the needle peripheral portion 4a, but the arrangement and shape of the microneedles and the body fluid flow path are not particularly limited.

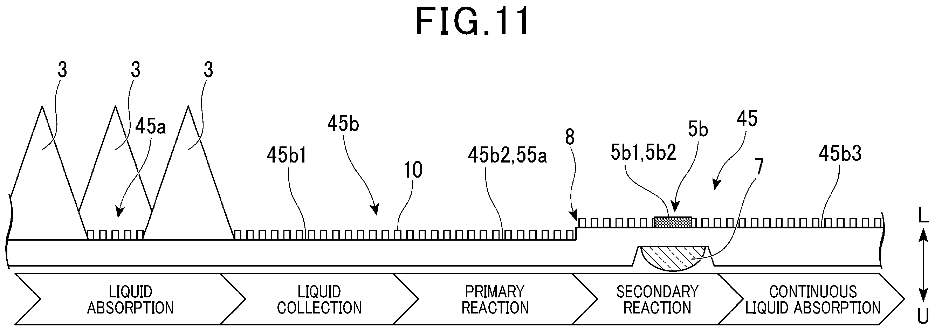

Fig. 11 is a schematic diagram showing the function of a body fluid flow path 45 according to yet another preferable embodiment of the present invention.

As shown in Fig. 11, the body fluid flow path 45 may be formed linearly on the lower surface of the substrate, and a needle peripheral portion 45a, a first downstream portion 45b1, a second downstream portion 45b2, and a third downstream portion 45b3 may be arranged in a straight line in order from the microneedle 3 side. In addition, in this case, the entire linear body fluid flow path may be formed in a shape in which the width increases toward the downstream side. In Fig. 11, the same reference numerals are given to the parts configured in the same manner as the examination device 1 according to the embodiment shown in Fig. 1.

As shown in Fig. 11, a conjugate unit 55a provided with the labeling substance may be formed in the second downstream portion 45b2. In a case where the conjugate unit 55a is formed in the second downstream portion 45b2, if the distance between the conjugate unit 55a where the primary reaction occurs and the color-producing unit 5b where the secondary reaction occurs becomes excessively short, it is preferable to form a step 8 between the conjugate unit 55a and the color-producing unit 5b on the body fluid flow path 45 so as to ensure sufficient time for labeling of the biomarker contained in the interstitial fluid. As a result of forming the step 8 in the body fluid flow path 45, the body fluid that has passed through the conjugate unit 55a can be brought into contact with the step 8, the flow velocity of the body fluid can be reduced, and the time for the body fluid to reach the color-producing unit 5b can be lengthened.

Note that the step 8 is formed such that the downstream section of the second downstream portion 45b2 in which the color-producing unit 55b is formed is located lower than the section in which the conjugate unit 55a is formed. However, the section of the second downstream portion 45b2 in which the color-producing unit 55b is formed may be located higher than the section in which the conjugate unit 55a is formed.

Furthermore, fibers such as cellulose, glass, cotton, or the like may be arranged in the body fluid flow path, in a similar manner to a conventional examination device using an immunoassay method. The location in the body fluid flow path in which the fibers are arranged is not particularly limited, and the fibers may be arranged in a portion or all of the body fluid flow path.

For example, when fibers are arranged in the linearly formed body fluid flow path 45 shown in Fig. 11 instead of the microprotrusions 10 or together with the microprotrusions 10, the fibers may be arranged in the first downstream portion 45b1 that collects the interstitial fluid. In addition, the fibers may be arranged in the section of the second downstream portion 45b2 in which the conjugate unit 55a is formed, in which the primary reaction occurs, or the fibers may be arranged in the section where the color-producing unit 5b is formed, in which the secondary reaction occurs. Also, the fibers may be arranged in the third downstream portion 45b3 that exclusively transports the interstitial fluid to the downstream side.

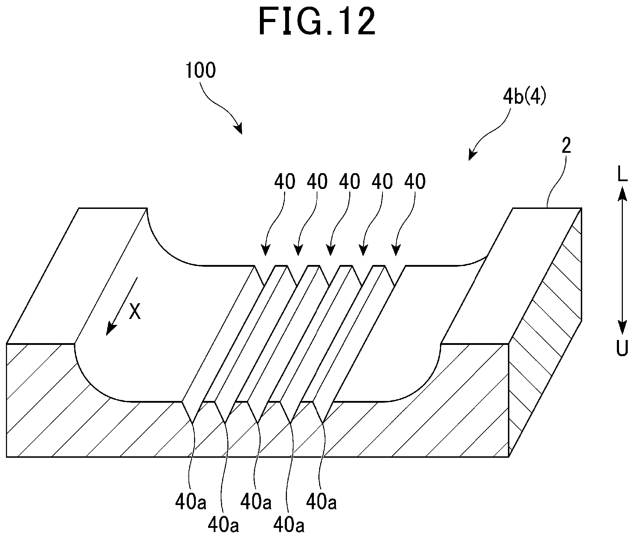

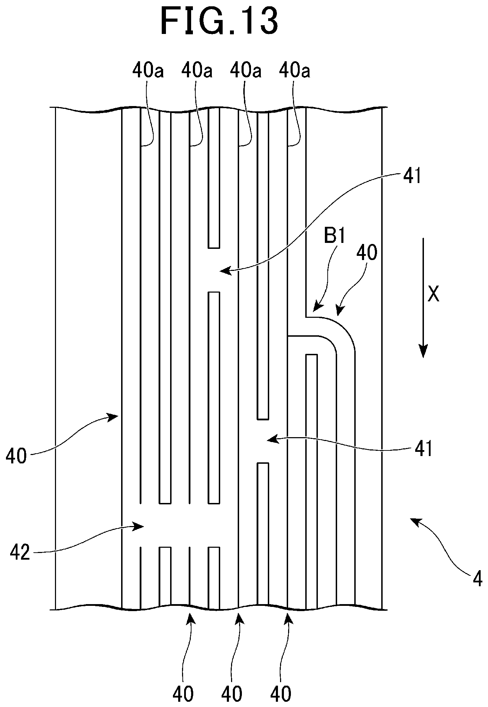

Fig. 12 is a schematic perspective view of the vicinity of the body fluid flow path 4 of an examination device 100 according to another preferable embodiment of the present invention, and Fig. 13 is a schematic bottom view of the vicinity of the transfer grooves 40 shown in Fig. 12.

Fig. 12 shows the downstream portion 4b, which is a portion of the body fluid flow path 4, including a vertical cross-section.

Note that Figs. 12 and 13 show different sections of the transfer grooves 40 in the extending direction of the downstream portion 4b, and the transfer grooves 40 shown in Fig. 12 represent a section immediately on the downstream side of the transfer grooves 40 shown in Fig. 13.

The examination device 100 according to the present embodiment is configured in the same manner to the examination device 1 according to the embodiment shown in Figs. 1 to 3, except for the aspects described below.

On the surface of the downstream portion 4b, a plurality of transfer grooves 40, which are an example of a "microstructure" for increasing the surface area of the downstream portion 4b and improving the wettability of the downstream portion 4b, are formed.

In Figs. 12 and 13, the bottom portion of each transfer groove 40 is indicated by reference sign 40a.

Each transfer groove 40 is formed in the direction in which the interstitial fluid is desired to flow within the downstream portion 4b, that is, toward the downstream side. The transfer grooves 40 extend parallel to each other on the surface of the downstream portion 4b. As a result of forming the transfer grooves 40 on the surface of the downstream portion 4b in this way, the surface area of the downstream portion 4b can be increased, and the transport speed of the interstitial fluid can be improved.

The width of each transfer groove 40 is preferably in a range of 100 nm or more and less than 1000 nm.

As shown in Fig. 13, the transfer grooves 40 branch at a branch point B1 of the downstream portion 4b. In this way, as a result of increasing the number of transfer grooves 40 toward the downstream side of the downstream portion 4b, the volume of the surface of the downstream portion 4b is increased, the transport force for the interstitial fluid produced by the downstream portion 4b and the flow velocity of the interstitial fluid are increased, and the deceleration of the interstitial fluid on the downstream side can be suppressed.

In the downstream portion 4b, two adjacent transfer grooves 40 are connected to each other by connecting grooves 41 and 42 extending in a direction orthogonal to the direction X in which the body fluid flows. As a result, the interstitial fluid can be stably transported.

Note that the connecting groove 41 connects two adjacent transfer grooves 40, and the connecting groove 42 connects three adjacent transfer grooves 40, but the number of transfer grooves 40 connected by a single connecting groove is not limited to two or three. Therefore, a configuration is possible in which four or more adjacent transfer grooves 40 are connected by a single connecting groove.

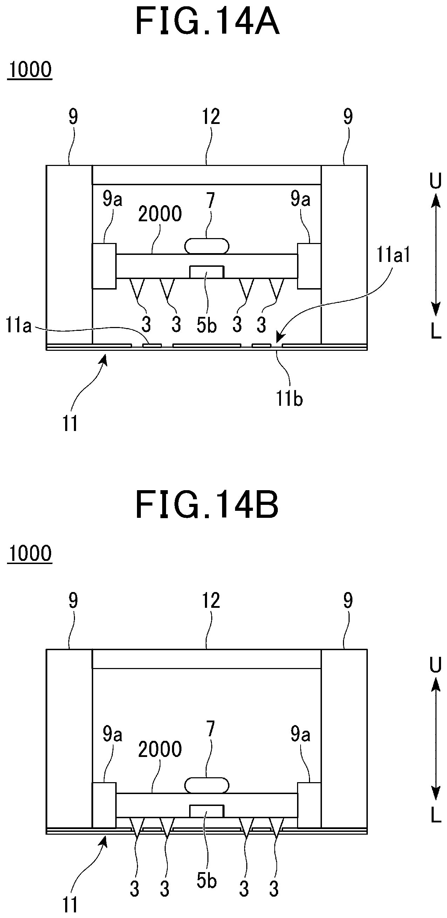

Fig. 14A is a schematic side view showing the vertical position of a substrate 2000 in a standard state of an examination device 1000 according to still another preferable embodiment of the present invention, and Fig. 14B is a schematic side view showing the vertical position of the substrate 2000 of the examination device 1000 in a piercing state.

The examination device 1000 according to the present embodiment is configured in the same manner as the examination device 1 according to the embodiment shown in Figs. 1 to 3, except for the aspects described below.

The examination device 1000 does not have the fixing seal 6, and includes a pair of substrate support portions 9 that support the substrate 2000, and a connecting portion 12 that connects the pair of substrate support portions 9.

A through-hole (not shown) that penetrates in the vertical direction of the connecting portion 12 is formed in the portion located above the optical system 7, and the color-producing unit 5b can be viewed through the through-hole and the optical system 7.

Each substrate support portion 9 has a slide portion 9a that is slidable in the vertical direction. The substrate 2000 is attached to the slide portions 9a of the pair of substrate support portions 9.

A protective film 11 that protects the microneedles 3 is provided on the tip end side, that is, below the microneedles 3. The left and right end portions (in the drawing) of the sheet surface of the protective film 11 are fixed to the lower surfaces of the pair of substrate support portions 9.

The protective film 11 includes a first layer 11a fixed to the pair of substrate support portions 9, and a second layer 11b attached to the lower surface of the first layer 11a. An adhesive layer is formed on the lower surface of the second layer 11b, but the presence or absence of the adhesive layer is not particularly limited. Also, it is not always necessary for the protective film 11 to include both the first layer 11a and the second layer 11b, and the protective film 11 may include only one of the first layer 11a and the second layer 11b. Moreover, the protective film 11 may include one or more additional layers in addition to the first layer 11a and the second layer 11b. In other words, the number of layers included in the protective film 11 is not particularly limited. In addition, as the function of the layer added to the protective film 11, it is possible to consider a function such as an improvement of the permeability of body fluid, an adhesion function, or the like, but the function is not limited thereto.

The first layer 11a plays the role of stretching the second layer 11b. Through-holes 11a1 that penetrate in the vertical direction are formed in the sections of the first layer 11a located below each microneedle 3.

The second layer 11b is configured to be pierceable by the microneedles 3, and plays the role of stretching the skin and protecting the microneedles 3 when piercing the skin.

The substrate 2000 is located at the vertical position shown in Fig. 14A in the standard state before piercing.

In a state where the lower surfaces of the pair of substrate support portions 9 and the protective film 11 are in contact with the skin, by pressing the substrate 2000 downward and sliding the substrate 2000 to the vertical position shown in Fig. 14B, the microneedles 3 are capable of piercing the skin (piercing state).

As shown in Fig. 14B, in the pierced state in which the microneedles 3 are piercing the skin, each microneedle 3 passes through the second layer 11b via the through-hole 11a1 and pierces the skin.

In this way, by piercing the skin through the protective film 11 in a state where the lower surface of the protective film 11 is in contact with the skin, the skin can be pierced while suppressing deformation of the skin, and interstitial fluid can be collected with minimal invasion.

The thickness of the protective film 11 is not particularly limited as long as the thickness is less than the height dimension of the microneedles 3, and can be set as appropriate. For example, in a case where the height of the microneedles 3 is in a range of 100 $\mu$m or more and 2 mm or less as described above, the thickness is preferably 50 $\mu$m or more and 1,950 $\mu$m or less, depending on the height of the microneedles 3.

The materials of the first layer 11a and the second layer 11b are not particularly limited, but for example, titanium, stainless steel, polyglycolic acid, polycarbonate, cyclic olefin copolymer (COC), cyclic olefin polymer, polyether ether ketone (PEEK), alginate, chitin, chitosan, collagen, hydroxypropyl cellulose, pullulan, and the like can be used.

The protective film 11 preferably includes, as the first layer 11a, the second layer 11b, or another layer, a layer that is permeable to body fluid such as interstitial fluid. As a result, the amount of body fluid that can be collected from the skin can be increased. Examples of materials permeable to body fluid such as interstitial fluid include nitrocellulose, polyvinylidene fluoride, nylon, polyether sulfone, and the like.

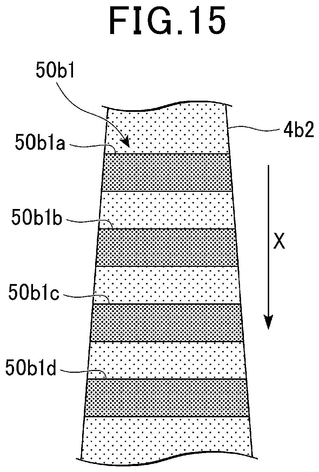

Fig. 15 is a schematic bottom view of the vicinity of a test unit 50b1 of an examination device capable of multi-item detection according to an embodiment of the present invention.

In the present embodiment, the same reference signs are used for the second downstream portion, which is configured in the same manner as the embodiment shown in Fig. 1, and the descriptions thereof are omitted.

The test unit 50b1 of the present embodiment includes, in the second downstream portion 4b2, four color-producing regions 50b1a to 50b1d arranged along the direction X in which the body fluid flows. In the four color-producing regions 50b1a to 50b1d, a single type of first antibody that specifically captures a mutually different antigen is provided in each region, which makes it possible to detect a total of four biomarkers. Hereinafter, the process from the collection of interstitial fluid to the visualization of the presence of the four biomarkers will be described.

In the conjugate unit formed in the first downstream portion 4b1, four types of labeling substances that bind one-to-one to each of the four types of biomarkers are mixed. The colors of the four types of labeling substances may be the same as each other, or may be different from each other.

When interstitial fluid is collected by the piercing of the microneedles 3 and transported to the first downstream portion 4b1 through the needle peripheral portion 4a, the four types of labeling substances in the conjugate unit disperse in the interstitial fluid. Then, the four types of labeling substances bind to the biomarkers that may be contained in the interstitial fluid while mixing with the interstitial fluid and flowing through the first downstream portion 4b1 and the second downstream portion 4b2 (the section further on the upstream side than the position where the test unit 50b1 is formed). As a result, up to four types of complexes are formed.

In each of the four color-producing regions 50b1a to 50b1d, a first antibody that specifically captures one of the four types of complexes is provided, and up to four types of complexes are captured in the color-producing regions 50b1a to 50b1d. As a result, the biomarker to which the labeling substance is bound is concentrated in up to four color-producing regions 50b1a to 50b1d, which becomes colored to an intensity that is visible through the optical system 7.

In this way, the four color-producing regions 50b1a to 50b1d can visualize the presence of mutually different biomarkers. Note that, in terms of the arrangement of the color-producing regions 50b1a to 50b1d in the second downstream portion 4b2, it is preferable to arrange the color-producing regions that visualize biomarkers contained in a small amount in the body fluid on the downstream side, and arrange the color-producing regions that visualize biomarkers contained in a large amount in the body fluid on the upstream side.

This lengthens the transport distance from when the biomarker contained in a small amount reaches the conjugate unit to when the biomarker reaches the corresponding color-producing region, ensuring sufficient a binding time (binding opportunity) between the biomarker contained in a small amount and the labeling substance, which enables the color production due to the biomarker contained in a small amount to be increased.

In addition, as described in detail below in Example 3, each color-producing region of the test unit may be formed in a region on the inner side of a groove (not inside the groove) extending in a closed loop shape (such as a circular shape or a rectangular shape) formed on the surface (bottom surface) of the body fluid flow path, such as the second downstream portion 4b2.

When the amount of body fluid transported through the body fluid flow path increases, and the body fluid crosses the groove structure extending around the color-producing region so as to surround the color-producing region, and reaches the color-producing region on the inner side, the biomarker that may be contained in the body fluid and the labeling substance bound to the biomarker are captured in the color-producing region and develop color, such that the biomarker is detected.

Furthermore, the number of types of antibodies and the number of color-producing regions are not limited to four, and may be three or less, or five or more.

In addition, in the present embodiment, as shown in Fig. 15, although the four color-producing regions 50b1a to 50b1d are arranged along the extending direction of the body fluid flow path (direction of arrow X), for example, a plurality of color-producing regions may be arranged in the width direction of the body fluid flow path (direction substantially orthogonal to arrow X).

Also, in an embodiment capable of multi-item detection, instead of the microprotrusions 10, the transfer grooves 40 and the connecting grooves 41 shown in Fig. 13 may be formed in the downstream portion 4b. Even in the case of multi-item detection, it is desirable that the number of transfer grooves 40 increases toward the downstream side of the body fluid flow path 4.

In addition, in the present embodiment, although the microneedles 3, the body fluid flow path 4, and the immunoassay unit including the test unit 50b1 are arranged on the lower surface 2a (the surface on the one side) of the substrate 2, a through-hole that penetrates the substrate in the vertical direction may be formed, and the through-hole may be used as the body fluid flow path, such that the body fluid collected by the microneedles 3 on the lower surface of the substrate is transported to the upper surface of the substrate, and an immunoassay unit including a test unit capable of multi-item detection may be provided on the upper surface side.

Furthermore, in the present embodiment, fibers such as cellulose, glass, cotton, or the like may be arranged in a portion or all of the body fluid flow path 4.

[Examination Method]

An examination method of the present invention includes: a step of piercing skin to collect a body fluid with a microneedle of an examination device, the examination device being provided with, on a surface on one side of a substrate, the microneedle, a body fluid flow path that transfers the body fluid, and an immunoassay unit that detects a biomarker using an antigen-antibody reaction; a step of transferring the collected body fluid through the body fluid flow path (or in other words, on the body fluid flow path); and a step of detecting, using the immunoassay unit, a biomarker contained in the body fluid that has been transported through the body fluid flow path.

The examination method of the present embodiment is a method of testing the presence or absence of a biomarker using the examination device according to the embodiments shown in Figs. 1 to 15, or an examination device described later. For this reason, in the examination method of the present embodiment, as described above, the skin is pierced with the microneedle, and the body fluid, which travels along the needle surface or passes through a hollow section inside the needle, is transported through the body fluid flow path, and during the transport, the biomarker that may be contained in the body fluid is detected by the immunoassay unit located in the body fluid flow path.

The immunoassay unit of the examination device used in the examination method may have a color-producing unit as described above, and may have a test unit that visualizes the presence of the biomarker in the body fluid. The test unit may include a plurality of color-producing regions that visualize the presence of mutually different biomarkers (in other words, the type of biomarker visualized may be different among the plurality of color-producing regions).

In this case, the color-producing region may be formed in a region inside a groove structure formed on the surface of the body fluid flow path and extending in a closed loop shape (such as a circular shape or rectangular shape), as in Example 3 described later. The plurality of color-producing regions may be located inside a single groove structure, or each color-producing region may have a groove structure on the outer side.

Antibodies that specifically capture mutually different biomarkers may be provided in each of the plurality of color-producing regions (in other words, the type of antibody may be different for each color-producing region). This allows different biomarkers to be captured and visualized (color detected with the naked eye or an image sensor) and detected for each color-producing region.

The present invention is not limited to the embodiments described, and various modifications are possible within the scope of the invention described in the claims, and it goes without saying that such modifications are also included within the scope of the present invention.

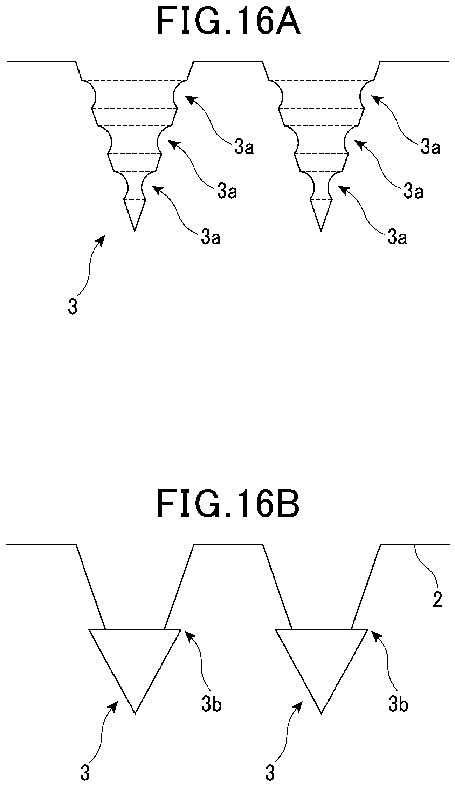

For example, in the embodiment shown in Figs. 1 to 3, fine unevenness is formed on the surface of the needle 3 by oxygen plasma treatment, but as shown in Fig. 16A, horizontal grooves 3a extending in the circumferential direction may be formed on the surface of the substantially conical microneedles 3, or as shown in Fig. 16B, the microneedles 3 may be formed in an arrowhead shape in which a portion of the microneedles 3 in the height direction protrudes toward the outer side in the radial direction.

As a result of forming unevenness such as the horizontal grooves 3a or the protruding portions 3b on the microneedle 3, the wettability of the microneedle 3 can be improved, and when piercing the skin, a partial gap is formed between the microneedle 3 and the skin, making it easier to supply the interstitial fluid that has seeped out from the skin to the body fluid flow path. Note that, in the microneedle 3 on which the horizontal grooves 3a are formed, when viewed from inside a horizontal groove 3a, the corner portion which is the entrance of the horizontal groove 3a is a convex portion, and in the arrowhead-shaped microneedle 3, a recess exists above the protruding portion 3b.

When forming the horizontal grooves 3a on the microneedle 3, the horizontal grooves 3a can be formed by inserting a trapezoidal tool, which has protruding shapes corresponding to the horizontal grooves 3a on the left and right, into the substantially trapezoidal space between adjacent microneedles 3 in a side view, and then moving the tool back and forth in the depth direction of the drawing. Similarly, in a case where the microneedle 3 is formed with an arrowhead shape, the microneedle 3 can be formed by moving a trapezoidal tool, which has protruding shapes on the left and right that cut the base portion of the needle 3, back and forth. Instead of a tool, it is also possible to form the horizontal grooves 3a or the protruding portions using, for example, a semiconductor lithography technique.

Furthermore, in the embodiment shown in Figs. 12 and 13, the transfer grooves 40 are formed in the body fluid flow path 4 as an example of the microstructure, but the body fluid flow path 4 may also be formed in a shape in which the width dimension increases toward the downstream side. Moreover, as shown in Fig. 10, the body fluid flow path may be formed in a shape in which a plurality of repeating units U, each having a shape in which the width dimension increases toward the downstream side, are connected in the extending direction of the body fluid flow path. As a result of such a configuration, the flow velocity of the interstitial fluid can be increased.

In addition, the configuration related to the protective film 11 and the pair of substrate support portions 9 shown in Fig. 14 may be applied to the embodiment shown in Figs. 12 and 13.

Also, in the embodiment shown in Figs. 1 to 3 and the embodiment shown in Fig. 10, the downstream portion 4b has a shape in which the width increases toward the downstream side, but at least a portion of the downstream portion 4b in the extending direction may be configured such that the depth of the downstream portion 4b becomes deeper toward the downstream side. This means that the vertical position of the upper surface of the downstream portion 4b shifts upward (the amount of recess toward the upper side of the substrate 2 increases), and the height of the wall portions defining both side portions of the downstream portion 4b increases toward the downstream side. As a result of such a configuration, the surface area of the downstream portion 4b can be increased, and the flow velocity of the interstitial fluid flowing through the downstream portion 4b can be increased.

Examples

The present invention will now be described in more detail by way of the following examples, but the present invention is not limited to the following examples.

<Example 1: Preparation of Microstructure for Improving Wettability (1)>

(Preparation of Microstructure)

First, a pattern for forming fine unevenness was transferred onto the surface of an aluminum plate (2 x 2 x 0.014 mm, purity 99.99%) using a 200 nm protrusion period structure mold, and anodic oxidation was performed in the following electrolytic solution to obtain a nanopore structure with a 200 nm pore period.

The electrolysis conditions were as follows.

| | |
|---|---|
| Electrolyte composition | : Phosphoric acid 5.0 g/L |
| Electrolysis voltage | : 100 V constant voltage |
| Electrolysis temperature | : 0°C |
| Electrolysis time | : 10 minutes |

Next, etching treatment was performed under the following conditions to arrange the pore structure.

| | |
|---|---|
| Etching solution composition | : Phosphoric acid 50 g/L |
| Etching solution temperature | : 30°C |
| Etching time | : 10 minutes |

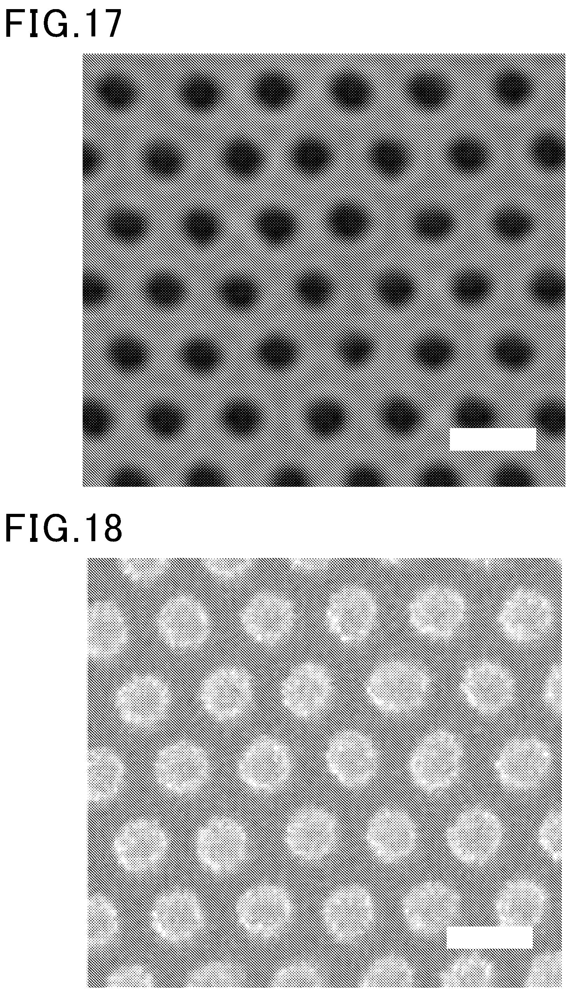

A high-magnification image of the mold structure obtained by the etching treatment, having a pore period of 200 nm, a pore diameter of 150 nm, and a pore length of about 400 nm, is shown in Fig. 17. The scale bar shown in FIG. 17 is 200 nm.

The simple mold obtained in this way was washed with water and dried, then a substrate formed with a nanopore structure was mounted, and methyl methacrylate (MMA) containing 10 wt% polyethylene glycol and 5 wt% benzoyl peroxide was applied dropwise onto the nanopore structure under vacuum to fill the pores with MMA and coat the pore structure substrate. Next, polymerization was performed by heating at 40°C for 48 hours, which produced PMMA. The PMMA-coated substrate formed with a nanopore structure was removed from the simple mold and immersed in a phosphoric acid-chromic acid mixed solution (35 mL of 85% phosphoric acid + 20 g/L chromium trioxide solution) at 30°C for 3 hours to selectively dissolve and remove the alumina, which allowed a structure with a pillar diameter of 150 nm, pillar height of 400 nm, and pillar-to-pillar spacing of 200 nm to be prepared. A high-magnification image of the obtained microstructure (flow path structure) is shown in Fig. 18. The scale bar shown in Fig. 18 is 200 nm.

(Evaluation of Wettability)

As the evaluation method of the wettability, a contact angle meter (CA-X, manufactured by Kyowa Interface Science Co., Ltd.) was used to place 1 μL of water from a syringe onto the obtained microstructure, and the contact angle was measured. The measurement was performed 3 times, and the average value was taken as the contact angle of each sample. As a comparative example, PMMA containing 10% PEG without a microstructure was also measured. Table 1 below shows the measurement results of the contact angle.

[Table 1]

| Material (Pillars/No Pillars) | Contact Angle (°) |
| --- | --- |
| PMMA containing 10% PEG (with pillars) | 20.7 ± 1.6 |
| Comparative example: PMMA containing 10% PEG (no pillars) | 52.8 ± 2.2 |

As shown in Table 1, the flow path structure having pillars on the surface had a remarkably smaller water contact angle compared to the comparative example having no pillars.

<Example 2: Preparation of Microstructure for Improving Wettability (2)>

(Preparation of Microstructure)

First, a pattern for forming fine unevenness was transferred onto the surface of a titanium plate (2 x 2 x 0.014 mm, purity 99.99%) using a 200 nm protrusion period structure mold, and anodic oxidation was performed in the following electrolytic solution to obtain a nanopore structure with a 200 nm pore period.

The electrolysis conditions were as follows.

Electrolyte composition     : 0.5 M phosphoric acid + 0.14 M sodium fluoride
Electrolysis voltage        : The anodic oxidation voltage was continuously ramped from the open circuit potential to higher values, generally from 10 to 30 V at a rate of 0.1 V/sec, and then raised to 40 V
Electrolysis temperature:    40°C
Electrolysis time           : 45 minutes

Next, etching treatment was performed under the following conditions to arrange the pore structure.

Etching solution composition     : Phosphoric acid 50 g/L
Etching solution temperature     : 30°C
Etching time                     : 10 minutes

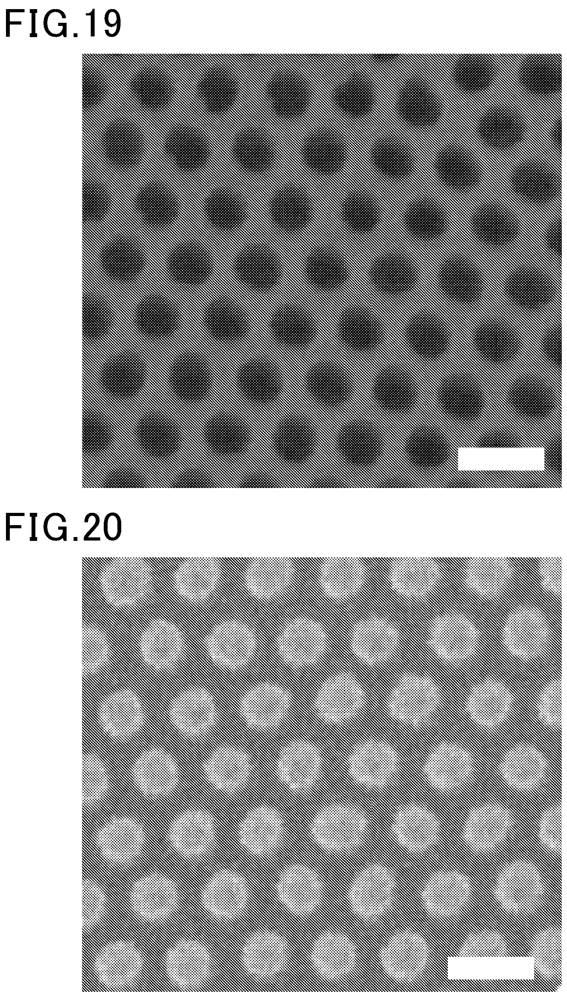

A high-magnification image of the mold structure obtained by the etching treatment, having a pore period of 200 nm, a pore diameter of 150 nm, and a pore length of about 1 μm, is shown in Fig. 19. The scale bar shown in Fig. 19 is 200 nm.

The simple mold obtained in this way was washed with water and dried, then a substrate formed with a nanopore structure was mounted, and methyl methacrylate (MMA) containing 10 wt% polyethylene glycol and 5 wt% benzoyl peroxide was applied dropwise onto the nanopore structure under vacuum to fill the pores with MMA and coat the pore structure substrate. Next, polymerization was performed by heating at 40°C for 48 hours, which produced PMMA. The PMMA-coated substrate formed with a nanopore structure was removed from the simple mold and immersed in a phosphoric acid-chromic acid mixed solution (35 mL of 85% phosphoric acid + 20 g/L chromium trioxide solution) at 30°C for 3 hours to selectively dissolve and remove the alumina, which allowed a microstructure (flow path structure) with a pillar diameter of 150 nm, pillar height of 1 $\mu$m, and pillar-to-pillar spacing of 200 nm to be prepared. Fig. 20 shows a high-magnification image of the obtained microstructure. The scale bar shown in Fig. 20 is 200 nm.

(Evaluation of Wettability)

As the evaluation method of the wettability, a contact angle meter (CA-X, manufactured by Kyowa Interface Science Co., Ltd.) was used to place 1 $\mu$L of water from a syringe onto the obtained microstructure, and the contact angle was measured. The measurement was performed 3 times, and the average value was taken as the contact angle of each sample. As a comparative example, PMMA containing 10% PEG without a microstructure was also measured.

[Table 2]

| Material (Pillars/No Pillars) | Contact Angle (°) |
| --- | --- |
| PMMA containing 10% PEG (with pillars) | 25.2 $\pm$ 2.1 |
| Comparative example: PMMA containing 10% PEG (no pillars) | 52.8 $\pm$ 2.2 |

As shown in Table 2, the flow path structure having pillars on the surface had a remarkably smaller water contact angle compared to the comparative example having no pillars.

<Example 3: Multi-item Detection of Biomarkers by Immunoassay Unit>

[Materials and Methods]

(Reagents)

The reagents shown in Table 3 below were used as reagents.

[Table 3]

| Product Name | Manufacturer | Model Number |
| --- | --- | --- |
| PBS | Wako Pure Chemical Industries | 166-23555 |
| Dedicated buffer (0.1% BSA/0.1% Triton/PBS) | Prepared in-house | - |
| Blocking buffer (10% BSA/PBS) | Prepared in-house | - |
| Anti-human IgG antibody [KT131] | abcam | ab211339 |
| Anti-human IgG antibody [RM116] | abcam | ab195574 |
| Anti-CRP-1 antibody [EPR14345-43] | abcam | ab185558 |
| Biotinylated human C reactive protein antibody [PTX-1] | abcam | ab108826 |
| Native human IgG protein (referred to as IgG below) | abcam | ab91102 |
| Recombinant human C-reactive protein (referred to as CRP below) | abcam | ab283925 |

(Materials)

The materials shown in Table 4 below were used as materials.

[Table 4]

| Product Name | Manufacturer | Model Number |
| --- | --- | --- |
| Colored beads (CMYKW) set | polysciences | 15706 |

(continued)

| Product Name | Manufacturer | Model Number |
|---|---|---|
| Immunochromatography flow path prototype | CSTEC | - |

(Preparation of Detection Antibody-Modified Colored Beads)

20 µL of colored beads C solution and 1 mg of anti-human IgG antibody [KT131], 20 µL of colored beads M solution and 1 mg of anti-human serum albumin antibody [15C7], and 20 µL of colored beads Y solution and 1 mg of anti-CRP-1 antibody [EPR14345-43] were each added to a dedicated buffer to make a total volume of 200 µL, and mixed by inversion at room temperature for 1 hour to prepare three types of detection antibody-modified beads.

(Direct Fixing of Capture Antibody or Fixing via Beads to Immunochromatography Flow Path)

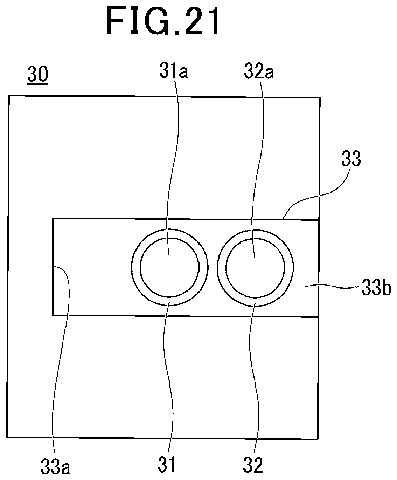

The capture antibody solutions were prepared by adding 1 mg of each of anti-human IgG antibody [RM116] and biotinylated human C reactive protein antibody [PTX-1] to a dedicated buffer to make a total volume of 500 µL.
Next, in an immunochromatography flow path prototype 30 provided with color-producing regions 31a and 32a inside two groove structures 31 and 32 extending in a circular shape (circular diameter 7 mm, groove diameter 0.75 mm, and groove depth 0.25 mm) formed on the bottom surface (surface) 33b of a body fluid flow path structure (recess structure formed in the substrate) 33 capable of autonomous liquid delivery, 10 µL of the prepared anti-human IgG antibody [RM116] antibody solution was added to one color-producing region 31a, and 10 µL of the prepared biotinylated human C reactive protein antibody [PTX-1] antibody solution was added to the other color-producing region 32a, and left to stand overnight at 4°C and dried. A top view of the obtained flow path prototype 30 is shown in Fig. 21.

(Preparation of Development Sample Solution)

The dedicated buffer and the solution containing the detection antibody-modified colored beads described above were mixed to prepare a total volume of 200 µL of development sample solutions containing IgG protein, Alb protein, and/or CRP protein in the compositions shown in Table 5 below. Sample A is a negative control containing none of the proteins.

[Table 5]

| Component | Sample A | Sample B | Sample C | Sample D |
|---|---|---|---|---|
| IgG (µg) | 0 | 400 | 400 | 0 |
| Alb (µg) | 0 | 400 | 400 | 400 |
| CRP (µg) | 0 | 400 | 0 | 400 |

(Immunochromatography Assay)

To a closed side end portion 33a, being one end portion of the body fluid flow path structure 33 of the immunochromatography flow path prototype 30, was added 50 µL of the development sample solution, and the solution was left to stand for 15 minutes, during which time the development sample solution was passively transported toward the other end side.

(Evaluation of Biomarker Detection)

The evaluation of whether the biomarker was detected in each color-producing region 31a and 32a was evaluated by visual observation, imaging using a desktop stereomicroscope (LPE-07W, manufactured by Sanwa Supply Inc.), and image analysis with ImageJ. In the visual evaluation, the presence or absence of coloring and the color were recorded. In the imaging using a desktop stereomicroscope (LPE-07W, manufactured by Sanwa Supply Inc.) and image analysis with ImageJ, a profile was created as a CMYK image from the captured data using ImageJ, and the presence or absence of C, M, and Y signal detection in the color-producing regions 31a and 32a was evaluated from an average signal value of 5 arbitrary points in the image.
The evaluation results are shown in Table 6 below. In the visual evaluation, "Present (color)" was recorded if coloring was present, and "Absent" if coloring was absent. In the signal detection, "All detected signals (CMY)" was recorded if a signal was detected, and "Absent" if none of the signals were detected.

[Table 6]

| Color-producing region 31a | Sample A | Sample B | Sample C | Sample D |
|---|---|---|---|---|
| Visual observation | None | Yes (blue) | Yes (blue) | None |
| Signal detection | None | C | C | None |
| Color-producing region 32a | Sample A | Sample B | Sample C | Sample D |
| Visual observation | None | Yes (yellow) | None | Yes (yellow) |
| Signal detection | None | Y | None | Y |

As a result, in Sample A, which contained none of the protein components, no coloring was observed visually, and no signal was detected by signal detection, whereas in Sample B, which contained all protein components, blue coloring was visually observed in the color-producing region 31a, yellow coloring was visually observed in the color-producing region 32a, and in the signal detection, a C signal was detected in the color-producing region 31a, and a Y signal was detected in the color-producing region 32a. Furthermore, in Sample C, which contained different protein components, blue coloring was visually observed in the color-producing region 31a, no coloring was visually observed in the color-producing region 32a, and in the signal detection, a C signal was confirmed in the color-producing region 31a, and no signal was confirmed in the color-producing region 32a. In Sample D, no coloring was visually observed in the color-producing region 31a, yellow coloring was visually observed in the color-producing region 32a, and in the signal detection, no signal was confirmed in the color-producing region 31a, and a Y signal was confirmed in the color-producing region 32a. Furthermore, the M signal was not detected in either of the color-producing region 31a and 32a, and no red coloring was visually observed, and therefore, Alb contained in samples B, C, and D was also not erroneously detected.

From the above, it was shown that the immunoassay unit of the present invention enables simultaneous detection and measurement of multiple biomarker items.

[Industrial Applicability]

According to the present invention, because the microneedle, the body fluid flow path, and the immunoassay unit are integrated on the surface on one side of the substrate, the number of special molds for the substrate can be reduced. As a result, manufacturing costs can be reduced, which enables industrial application.

[Reference Signs List]

1 Examination device
2 Substrate
2a Surface on one side of substrate (lower surface)
2b Surface on another side of substrate (upper surface)
3 Microneedle
3a Horizontal groove
3b Protruding portion
4 Body fluid flow path
4a Needle peripheral portion
4b Downstream portion
5 Immunoassay unit
5a Conjugate unit
5b Color-producing unit
5b1 Test unit
5b2 Control unit
6 Fixing seal
7 Optical system
9 Substrate support portion
10 Microprotrusion
11 Protective film

11a First layer
11b Second layer
11a1 Through-hole
13 Support
14 Frame
15 Well structure
20 Microprotrusion
30 Immunochromatography flow path prototype
31 Groove structure
31a Color-producing region
32 Groove structure
32a Color-producing region
33 Body fluid flow path structure
33a Closed side end portion
33b Bottom surface
40 Transfer groove
40a Bottom portion
41 Connecting groove
U Repeating unit

**Claims**

1. An examination device comprising:

   a substrate;
   a microneedle that pierces skin to collect a body fluid;
   a body fluid flow path that transfers the body fluid collected by the microneedle; and
   an immunoassay unit that detects, using an antigen-antibody reaction, a biomarker that may be included in the
   body fluid that has been transferred through the body fluid flow path, wherein
   the microneedle, the body fluid flow path, and the immunoassay unit are disposed on a surface on one side of the
   substrate.

2. The examination device according to claim 1, wherein
   a microstructure for improving wettability of the body fluid flow path is formed on a surface of the body fluid flow path.

3. The examination device according to claim 2, wherein
   the microstructure is a groove extending in an extending direction of the body fluid flow path.

4. The examination device according to claim 3, wherein
   a plurality of the grooves extend parallel to each other on a surface of the body fluid flow path.

5. The examination device according to claim 4, wherein
   a number of the grooves increases toward a downstream side of the body fluid flow path.

6. The examination device according to claim 5, wherein
   a connecting groove is formed that connects the plurality of grooves extending in the extending direction of the body
   fluid flow path to each other.

7. The examination device according to claim 6, wherein
   a width of the groove is in a range of 100 nm or more and less than 1,000 nm.

8. The examination device according to any one of claims 1 to 7, wherein
   at least a portion of the body fluid flow path has a shape in which a width increases toward a downstream side.

9. The examination device according to claim 8, wherein
   the body fluid flow path has a plurality of repeating units having the shape, that are connected in the extending direction
   of the body fluid flow path.

10. The examination device according to any one of claims 1 to 7, wherein
at least a portion of the body fluid flow path has a depth that becomes deeper toward a downstream side.

11. The examination device according to claim 2, wherein
the microstructure includes a plurality of microprotrusions provided on the body fluid flow path.

12. The examination device according to claim 11, wherein
the microprotrusions have a substantially conical shape, and when a height of the microprotrusions is a, a width dimension of a bottom surface portion of the microprotrusions is b, and an arrangement interval of the microprotrusions is c, the microprotrusions have a shape that satisfies the following formulas (1), (2), and (3).

$$b \leq 600 \text{ (unit: nm) ... (1)}$$

$$a/b \geq 0.3 \text{ ... (2)}$$

$$200 \leq c \leq 400 \text{ (unit: nm) ... (3)}$$

13. The examination device according to any one of claims 1 to 7, wherein unevenness is formed on a surface of the microneedle.

14. The examination device according to any one of claims 1 to 7, wherein

a protective film is provided on a tip end side of the microneedle, and
a thickness of the protective film is less than a height of the microneedle.

15. The examination device according to any one of claims 1 to 7, wherein
a section of the substrate located on another side of the immunoassay unit is formed to be transparent.

16. The examination device according to claim 15, wherein
a magnifying optical system is provided on another side of the section located on the other side of the immunoassay unit.

17. The examination device according to any one of claims 1 to 7, wherein
the surface on the one side of the substrate and a surface of the microneedle are formed of the same material, and a water contact angle on the surface on the one side is 45° or less.

18. The examination device according to any one of claims 1 to 7, wherein
the microneedle is a solid, hollow, porous, or hydrogel structure.

19. The examination device according to any one of claims 1 to 7, wherein
the body fluid flow path has a shape that surrounds the microneedle.

20. The examination device according to any one of claims 1 to 7, wherein

the immunoassay unit is provided with a test unit that visualizes a presence of the biomarker in the body fluid, and
the test unit is provided with a plurality of color-producing regions that visualize a presence of mutually different biomarkers.

21. The examination device according to claim 20, wherein

the color-producing regions are formed in a region on an inner side of a groove structure formed on a surface of the body fluid flow path and extending in a closed loop shape, and
antibodies that specifically capture mutually different biomarkers are provided in each of the plurality of color-producing regions.

22. An examination method, comprising:

a step of piercing skin to collect a body fluid with a microneedle of an examination device, the examination device being provided with, on a surface on one side of a substrate, the microneedle, a body fluid flow path that transfers the body fluid, and an immunoassay unit that detects a biomarker using an antigen-antibody reaction;

a step of transferring the collected body fluid through the body fluid flow path; and

a step of detecting, using the immunoassay unit, a biomarker contained in the body fluid that has been transported.

23. The examination method according to claim 22, wherein

the immunoassay unit is provided with a test unit that visualizes a presence of the biomarker in the body fluid, the test unit is provided with a plurality of color-producing regions that visualize a presence of mutually different biomarkers,

the color-producing regions are formed in a region on an inner side of a groove structure formed on a surface of the body fluid flow path and extending in a closed loop shape,

antibodies that specifically capture mutually different biomarkers are provided in each of the plurality of color-producing regions, and different biomarkers are captured and detected in each color-producing region by the antibodies.

# FIG.1

# FIG.2

# FIG.3

EP 4 786 981 A1

FIG.4

FIG.5

FIG.6

# FIG.7

# FIG.8A

# FIG.8B

# FIG.9A

# FIG.9B

FIG.10

# FIG.11

# FIG.12

# FIG.13

# FIG.14A

1000

# FIG.14B

1000

# FIG.15

# FIG.16A

# FIG.16B

## FIG.17

## FIG.18

FIG.19

FIG.20

# FIG.21

30

31a    32a

33

33b

33a    31    32

## INTERNATIONAL SEARCH REPORT

| | International application No. |
|---|---|
| | **PCT/JP2024/034349** |

### A. CLASSIFICATION OF SUBJECT MATTER

*G01N 33/543*(2006.01)i; *A61B 5/151*(2006.01)i; *A61B 5/157*(2006.01)i; *G01N 33/48*(2006.01)i; *G01N 33/50*(2006.01)i;
*G01N 33/53*(2006.01)i
FI: G01N33/543 521; G01N33/50 Z; G01N33/50 Q; G01N33/53 Z; G01N33/48 S; A61B5/157; A61B5/151

According to International Patent Classification (IPC) or to both national classification and IPC

### B. FIELDS SEARCHED

Minimum documentation searched (classification system followed by classification symbols)

G01N33/543; A61B5/151; A61B5/157; G01N33/48; G01N33/50; G01N33/53

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2021-506391 A (BRIGHTER AB PUBL) 22 February 2021 (2021-02-22) | 1, 18-19, 22 |
| | claims, paragraphs [0001], [0026]-[0032], [0038], fig. 1-15 | |
| Y | | 1-23 |
| Y | WO 2019/176126 A1 (THE UNIVERSITY OF TOKYO) 19 September 2019 (2019-09-19) | 1-23 |
| | paragraphs [0011]-[0019], [0025]-[0031], fig. 1-4 | |
| Y | WO 2019/117102 A1 (DENKA CO., LTD.) 20 June 2019 (2019-06-20) | 1-23 |
| | paragraphs [0013], [0023], [0026]-[0031], [0044], [0049]-[0053], [0081]-[0084] | |
| Y | JP 2009-541738 A (ÅMIC AB) 26 November 2009 (2009-11-26) | 1-23 |
| | abstract, claims, paragraphs [0038], [0077]-[0078] | |
| A | JP 2022-545144 A (LEKKOS, VASILEIOS) 26 October 2022 (2022-10-26) | 1-23 |
| | entire text, all drawings | |

✓ Further documents are listed in the continuation of Box C.    ✓ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 October 2024** | **29 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

| International application No. |
| --- |
| **PCT/JP2024/034349** |

| **C.** | **DOCUMENTS CONSIDERED TO BE RELEVANT** | |
| --- | --- | --- |
| Category* | Citation of document, with indication, where appropriate, of the relevant passages | Relevant to claim No. |
| A | WO 2023/042525 A1 (LINTEC CORPORATION) 23 March 2023 (2023-03-23)<br>entire text, all drawings | 1-23 |
| A | JP 2005-532151 A (ÅMIC AB) 27 October 2005 (2005-10-27)<br>entire text, all drawings | 1-23 |

Form PCT/ISA/210 (second sheet) (July 2022)

EP 4 786 981 A1

## INTERNATIONAL SEARCH REPORT
### Information on patent family members

| International application No. |
| --- |
| **PCT/JP2024/034349** |

| Patent document cited in search report | | | Publication date (day/month/year) | Patent family member(s) | | | Publication date (day/month/year) |
| --- | --- | --- | --- | --- | --- | --- | --- |
| JP | 2021-506391 | A | 22 February 2021 | US | 2020/0330008 | A1 | |
| | | | | claims, figures | | | |
| | | | | WO | 2019/121324 | A1 | |
| | | | | CN | 111526791 | A | |
| | | | | KR | 10-2020-0103023 | A | |
| WO | 2019/176126 | A1 | 19 September 2019 | US | 2020/0405235 | A1 | |
| | | | | abstract, fig. 1-4 | | | |
| | | | | EP | 3766422 | A1 | |
| | | | | CN | 111836582 | A | |
| | | | | KR | 10-2020-0132889 | A | |
| WO | 2019/117102 | A1 | 20 June 2019 | US | 2021/0181194 | A1 | |
| | | | | abstract, paragraphs [0022]-[0039] | | | |
| | | | | CN | 111465853 | A | |
| | | | | KR | 10-2020-0097264 | A | |
| JP | 2009-541738 | A | 26 November 2009 | US | 2010/0041154 | A1 | |
| | | | | abstract, claims | | | |
| | | | | WO | 2007/149043 | A1 | |
| | | | | CA | 2654931 | A1 | |
| | | | | CN | 101506657 | A | |
| JP | 2022-545144 | A | 26 October 2022 | US | 2022/0225902 | A1 | |
| | | | | all | | | |
| | | | | EP | 3965846 | A1 | |
| | | | | CN | 114126680 | A | |
| | | | | CA | 3140254 | A1 | |
| WO | 2023/042525 | A1 | 23 March 2023 | CN | 117897199 | A | |
| | | | | all | | | |
| | | | | KR | 10-2024-0056491 | A | |
| JP | 2005-532151 | A | 27 October 2005 | US | 2005/0042766 | A1 | |
| | | | | all | | | |
| | | | | CN | 1658972 | A | |

Form PCT/ISA/210 (patent family annex) (July 2022)

40

**EP 4 786 981 A1**

**Patent documents cited in the description**

- JP 2023163247 A
- JP 2024113244 A
- WO 2019118420 A